Europäisches Patentamt

European Patent Office

Office européen des brevets

(19)

(11) Publication number: **0 137 436**
**A2**

(12) # EUROPEAN PATENT APPLICATION

(21) Application number: **84111690.8**

(22) Date of filing: **29.09.84**

(51) Int. Cl.⁴: **A 61 K 7/18**

(30) Priority: **08.10.83 GB 8326974**

(43) Date of publication of application:
**17.04.85 Bulletin 85/16**

(84) Designated Contracting States:
**BE CH DE FR GB IT LI NL SE**

(71) Applicant: BEECHAM GROUP PLC
Beecham House Great West Road
Brentford Middlesex TW8 9BD(GB)

(72) Inventor: Duke, Susan Ann
6 Clifton Close Addlestone
Weybridge Surrey KT15 2EX(GB)

(72) Inventor: Pearl, Adrian Clive
1 Fairview Drive
Watford Hertfordshire(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) Oral hygiene compositon.

(57) A non-abrasive oral hygiene composition comprising an aqueous solution of alkali metal fluoride, alkali metal monofluorophosphate and orthophosphate anions, the latter having a concentration of at least 0.1mM in the aqueous solution, and the solution having a pH of from 7 to 11.

EP 0 137 436 A2

_1_

## ORAL HYGIENE COMPOSITION

The present invention relates to oral hygiene compositions having improved anticaries effect.

A variety of anticaries agents have hitherto been used in oral compositions especially dentifrices, including alkali metal fluorides and alkali metal monofluorophosphates. It has now been discovered that enhanced anticaries effect can be achieved when a combination of alkali metal fluoride, alkali metal monofluorophosphate and a dissolved orthophosphate are used within a certain pH range.

Accordingly the present invention provides a non-abrasive oral hygiene composition comprising an aqueous solution of alkali metal fluoride, alkali metal monofluorophosphate, and orthophosphate anions, the latter having a concentration of at least 0.1mM in the aqueous solution, and the solution having a pH of from 7 to 11.

Preferably the alkali metal fluoride is sodium fluoride.

Preferably the alkali metal monofluorophosphate is sodium monofluorophosphate.

Preferably from 10 to 90%, more preferably 40 to 80% of the fluorine is provided by the alkali metal fluoride and the remainder is provided by the alkali metal monofluorophosphate.

Suitably the orthophosphate anions are provided by trisodium orthophosphate, disodium hydrogen orthophosophate, tripotassium orthophosphate or dipotassium hydrogen orthophosphate, preferably by trisodium or disodium hydrogen orthophosphate. Other orthophosphate salts may be used in place of these or in combination with these but if less basic orthophosphates are employed it may be necessary to buffer the solution to achieve the desired pH.

Preferably the solution comprises at least 0.25m$\underline{M}$ dissolved orthophosphate anions, especially if the solution has pH from 7 to 8.

Preferably the aqueous solution has a pH from 7.25 to 10.5 more preferably up to about pH 8.5.

The compositions may optionally contain other agents known to enhance the anticaries effect of fluoride and monofluorophosphate, such as calcium glycerophosphate, this being incorporated in a weight ratio of up to 1:3, preferably 1:20 to 1:3 to the total weight of fluoride and monofluorophosphate salts.

- 3 -

The composition of the invention may be presented as a mouthwash or as gels, sweets, chewing gums etc or as a concentrate for dilution and subsequent use as a mouthwash. Gels, sweets and chewing gums would normally contain a solution of orthophosphate from 2 to 5 times more concentrated than the mouthwash since these compositions are diluted by a factor of up to 5 by saliva in the mouth.

When presented as mouthwashes the compositions will normally contain from 10 to 1000 ppm of fluoride , preferably 10 to 500 ppm and most preferably about 250 ppm. For other, more concentrated, presentations the maximum level of fluoride may be increased up to 5000 ppm.

Oral hygiene compositions according to the invention have a two-fold effect on caries - the fluoride agents cause formation of fluorohydroxy apatite in the enamel and thereby harden the teeth against caries, and they reduce plaque growth and increase plaque pH thereby reducing acid formation and hence reducing the severity of attack on the tooth enamel. Both these effects are enhanced by the orthophosphate.

Compositions according to the present invetion may be produced by conventional methods and may contain the usual optional, water-soluble, accessory ingredients such as sweetening agents, for example soluble saccharin, flavouring or colouring agents, preservative (e.g. sodium benzoate), silicones, alcohol, menthol, chlorophyll compounds (e.g. sodium copper chlorophyllin), antibacterial agents (e.g. chlorhexidine), anti-plaque agents, anti-calculus

- 4 -

agents, agents for sensitive dentine (e.g. strontium salts, formaldehyde), and agents which enhance the anti caries activity of fluorides.

The invention is illustrated by the following Examples.

| Mouthwash Formulae | Example 1 % w/v | Example 2 % w/v |
|---|---|---|
| 70% sorbitol | - | 5.00 |
| Propylene glycol | 5.00 | - |
| * Cremophor RH 40 (solubiliser) | 0.30 | - |
| Saccharin | 0.003 | 0.003 |
| ** Nipasept | 0.10 | 0.10 |
| *** Bronopol | 0.01 | 0.01 |
| Dyes (Blue No 1) | 0.001 | 0.001 |
| (Yellow No 10) | 0.003 | 0.003 |
| Flavours | 0.05 | 0.05 |
| Sodium fluoride | 0.032 | 0.032 |
| Sodium monofluorophosphate | 0.1 | 0.1 |
| Trisodium orthophosphate dodecahydrate | - | 0.15 |
| Disodium hydrogen phosphate dodecahydrate | 0.28 | 0.14 |
| Water | to 100% | |

* Glycerol polyethylene glycol oxystearate

** Mixture of sodium salts - Methylhydroxybenzoate
- Ethylhydroxybenzoate
- Propylhydroxybenzoate

*** 2-bromo-2-nitropropane-1,3-diol

Set A

## Claims

1.   A non-abrasive oral hygiene composition comprising an aqueous solution of alkali metal fluoride, alkali metal monofluorophosphate and orthophosphate anions, the latter having a concentration of at least 0.1mM in the aqueous solution, and the solution having a pH of from 7 to 11.

2.   A composition according to claim 1 containing from 10 to 5000ppm of fluoride ions in total.

3.   A composition according to claim 1 or 2, in which from 10 to 90% of the fluoride is provided by an alkali metal fluoride, and the remainder by an alkali metal monofluorophosphate.

4.   A composition according to any one of claims 1 to 3, in which orthophosphate anions are provided by trisodium orthophosphate, disodium hydrogen orthophosphate, tripotassium orthophosphate or dipotassium hydrogen orthophosphate.

5.   A composition according to any one of claims 1 to 4 in which the aqueous solution comprises at least 0.25mM dissolved orthophosphate anions.

6.   A composition according to any one of claims 1 to 5, in which the aqueous solution has a pH of from 7.25 to 10.5.

7.    A composition according to any one of claims 1 to
6 when presented as a mouthwash.

8.    A composition according to claim 7 containing from
10 to 1000 ppm of fluoride.